# FASCICULE DE BREVET EUROPEEN

(11) **EP 4 203 969 B1**
(45) Date de publication et mention de la délivrance du brevet: **08.10.2025**
(21) Numéro de dépôt: 21770056.6
(22) Date de dépôt: 26.08.2021
(51) Int. Cl.: A61K 31/7024, A61K 9/00, A61L 15/28, A61L 15/44, A61P 17/02

(54) **SUCROSE OCTASULFATE POUR SON UTILISATION POUR AUGMENTER L'OXYGENATION DE LA PEAU LORS DU TRAITEMENT DES PLAIES ISCHEMIQUES**
SACCHAROSEOCTASULFAT ZUR VERWENDUNG BEI DER ERHÖHUNG DER SAUERSTOFFANREICHERUNG DER HAUT BEI DER BEHANDLUNG ISCHÄMISCHER WUNDEN
SUCROSE OCTASULFATE FOR USE IN INCREASING SKIN OXYGENATION DURING TREATMENT OF ISCHEMIC WOUNDS

(30) Priorité: 26.08.2020 FR 2008725
(43) Date de publication de la demande: 05.07.2023
(73) Titulaire: URGO RECHERCHE INNOVATION ET DEVELOPPEMENT, 21300 Chenôve (FR)
(72) Inventeur: BOHBOT, Serge, 92300 LEVALLOIS-PERRET (FR); LÁZARO MARTÍNEZ, José Luis, 28660 Boadilla del Monte (ES)
(74) Mandataire: Plasseraud IP
(86) Numéro de dépôt international: PCT/FR2021/051487
(87) Numéro de publication internationale: WO 2022/043636

(56) Documents cités:
- FR-A1- 2 993 781
- FR-A1- 3 066 390
- US-A1- 2017 182 125
- DISSEMOND JOACHIM ET AL: "Sucrose Octasulfat - Evidenz in der Behandlung chronischer Wunden", HAUTARZT, SPRINGER VERLAG, BERLIN, DE, vol. 71, no. 10, 7 July 2020 (2020-07-07), pages 791 - 801, XP037256176, ISSN: 0017-8470, [retrieved on 20200707], DOI: 10.1007/S00105-020-04637-9
- LÁZARO-MARTÍNEZ JOSÉ LUIS ET AL: "Increasing Transcutaneous Oxygen Pressure in Patients With Neuroischemic Diabetic Foot Ulcers Treated With a Sucrose Octasulfate Dressing: A Pilot Study", INTERNATIONAL JOURNAL OF LOWER EXTREMITY WOUNDS, 28 August 2020 (2020-08-28), XP055816768, Retrieved from the Internet <URL:https://journals.sagepub.com/doi/pdf/10.1177/1534734620952244> [retrieved on 20210622]

## Description

La présente invention concerne un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, *i.e.* la cicatrisation des plaies ischémiques, ledit oligosaccharide étant le sucrose octasulfate.

### Contexte de l'invention

La cicatrisation d'une plaie est un phénomène biologique naturel, les tissus mammifères étant capables de réparer des lésions localisées par des processus de réparation et de régénération qui leur sont propres.

La rapidité et la qualité de la cicatrisation d'une plaie dépendent de l'état général de l'organisme atteint, de l'étiologie de la plaie, de l'état et de la localisation de la plaie, et de la survenue ou non d'une infection, ainsi que des facteurs génétiques prédisposant ou non à des troubles de la cicatrisation.

La cicatrisation naturelle d'une plaie se déroule principalement selon trois phases successives, chacune de ces phases étant caractérisée par des activités cellulaires spécifiques qui font progresser le processus de réparation selon des séquences chronologiques précises : la phase inflammatoire, la phase de granulation (ou phase proliférative), et la phase de maturation.

La première phase, la phase inflammatoire, débute dès la rupture des vaisseaux sanguins qui déclenche la formation d'un caillot (coagulation du sang) principalement composé de fibrine et de fibronectine, et qui va constituer une matrice provisoire. Cette matrice comble en partie la lésion et va permettre la migration au sein de la zone lésée des cellules inflammatoires recrutées pour assurer la détersion de la plaie. Les plaquettes présentes vont également libérer des facteurs (par exemple cytokine, facteurs de croissance) permettant le recrutement des cellules de la cicatrisation comme les cellules inflammatoires (les polynucléaires neutrophiles et les macrophages), les fibroblastes et les cellules endothéliales.

La seconde phase correspond au développement du tissu de granulation. On observe d'abord une colonisation de la blessure par prolifération des fibroblastes. Puis, la migration des cellules endothéliales à partir des vaisseaux sains va permettre la formation de nouveaux vaisseaux sanguins (néovascularisation), ou angiogénèse, du tissu lésé. Cette étape d'angiogenèse est fondamentale pour amorcer la cicatrisation. Dans le tissu de granulation, les fibroblastes sont activés et vont se différencier en myofibroblastes présentant des propriétés contractiles importantes, générées par les microfilaments d'actine, permettant la contraction de la plaie.

La troisième phase du processus de réparation, la maturation, s'accompagne d'un remodelage du tissu de granulation. Une partie de la matrice extracellulaire est digérée par des protéases (essentiellement des métallo-protéases matricielles (MMP) et des élastases), et on observe une réorganisation progressive de la matrice extracellulaire. Progressivement, le collagène de type III, majoritaire dans le tissu de granulation, est remplacé par le collagène de type I, principal composant matriciel du derme. A la fin de la phase de maturation, les fibroblastes, myofibroblastes et cellules vasculaires voient leur prolifération et/ou leur activité réduites. Puis les cellules excédentaires meurent par apoptose. Parallèlement au remodelage de la matrice extracellulaire et à l'apoptose des cellules excédentaires, l'état inflammatoire diminue progressivement. Cette phase est la plus longue : au bout d'un an environ, la cicatrice se remodèle, elle n'est plus rouge, ni rigide, ne provoque plus de douleur et elle s'aplanie.

Néanmoins, certains types de plaies ne cicatrisent pas correctement, les 3 étapes clés du processus se déroulant de manière anormale et ce, malgré la mise en place des meilleures conditions physico-chimiques et biologiques possibles. En effet la rapidité et la qualité de la cicatrisation d'une plaie dépendent de facteurs intrinsèques et extrinsèques. Ce processus de réparation peut donc être anormalement prolongé selon :
- l'étiologie de la plaie ;
- son état et sa localisation ;
- la survenue d'une infection causée par la présence de certains agent infectieux comme *Staphylococcus aureus* ou *Pseudomonas aeruginosa ;*
- l'existence d'une pathologie préexistante (comme le diabète, une déficience immunitaire, une insuffisance veineuse, etc...) ;
- l'environnement extérieur ; ou
- des facteurs génétiques prédisposant ou non à des troubles de la cicatrisation.

Parmi ces plaies qui ne cicatrisent pas correctement on retrouve les plaies ischémiques, notamment les ulcères et les escarres. Plus particulièrement, de telles plaies sont l'ulcère de jambe artériel, l'ulcère de jambe mixte, l'escarre de décubitus, et l'ulcère du pied du diabétique neuro-ischémique. Les plaies qui ne cicatrisent pas correctement sont plus particulièrement les plaies ischémiques de grade 2 et/ou 3.

Le traitement des plaies ischémiques a déjà été décrit, notamment le traitement du pied du diabétique. Par exemple, le brevet FR3066390 décrit l'utilisation de composés oligosaccharides pour traiter l'ulcère du pied du diabétique artériopathique. Edmonds *et al.* décrivent également l'utilisation de sucrose octasulfate pour le traitement de l'ulcère du pied du diabétique neuro-ischémique (Lancet Diabetes Endocrinol 2017), de même que Lazaro-Martinez *et al.* décrivent l'utilisation d'un pansement TLC-NOSF pour le traitement de l'ulcère du pied du diabétique neuro-ischémique (Journal of Wound Care, Volume 28, June 2019).

Les directives internationales recommandent également l'utilisation d'un pansement TLC-NOSF (Technology Lipido-Colloïd - Nano-OligoSaccharide Factor) pour le traitement de l'ulcère du pied du diabétique neuro-ischémique (Rayman G, et al., Guidelines on use of interventions to enhance healing of chronic foot ulcers in diabetes (IWGDF 2019 update). Diabetes Metab Res Rev. 2020;36 Suppl 1:e3283, ou encore National Institute for Health and Care Excellence. UrgoStart for treating leg ulcers and diabetic foot ulcers. January 2019, https://www.nice.org.uk/guidance/mtg421.

Les composés oligosaccharidiques ont également déjà été décrits pour leur utilisation dans l'activation de l'angiogénèse, par exemple dans le brevet FR3043556.

Les composés oligosaccharidiques ont aussi été décrits pour le traitement de l'ulcère du pied du diabétique neuropathique (Richard et al., Journal of Wound Care, Vol 21, N°3, March 2012).

Cependant de nouveaux traitements des plaies sont toujours souhaités, de même qu'une meilleure compréhension du phénomène de cicatrisation.

Une bonne oxygénation des plaies est par exemple un facteur favorisant la cicatrisation. Au contraire, l'ischémie retarde la cicatrisation ou l'empêche même dans les cas les plus critiques menant à la nécrose.

La présente invention s'intéresse ainsi plus particulièrement à l'augmentation de l'oxygénation de la peau pour le traitement des plaies ischémiques de grade 2 et/ou 3, notamment pour permettre une meilleure cicatrisation des plaies ischémiques.

Moon KC, Chung HY, Han SK, Jeong SH, Dhong ES. ont démontré une augmentaiton locale de la TcPO₂ chez des patients ayant un pied diabétique ischémique, après l'injection de la fraction stromale du tissu adipeux (SVF cells, adipose-derived stromal vascular fraction cells), dans l'étude « Possibility of Injecting Adipose-Derived Stromal Vascular Fraction Cells to Accelerate Microcirculation in Ischemic Diabetic Feet: A Pilot Study. Int J Stem Cells. 2019;12(1):107-13 ». Les auteurs ont montré une augmentaiton de la TcPO₂ jusqu'à 4 semaines puis une légère diminution.

Au contraire, la présente invention s'intéresse à l'augmentation de l'oxygénation de la peau à l'aide d'un oligosaccharide polysulfaté synthétique ayant 1 à 4 unités oses, ses sels, ou ses complexes. De tels composés présentent notamment l'avantage d'augmenter progressivement l'oxygénation (*i.e.* la valeur de la TcPO₂) jusqu'à la fermeture de la plaie et/ou le traitement de l'ischémie.

### Exposé de l'invention

L'invention est telle que définie dans les revendications 1 à 10.

L'invention concerne ainsi un oligosaccharide polysulfaté synthétique ayant 1 à 4 unités oses, ses sels, ou ses complexes, pour son utilisation pour augmenter l'oxygénation de la peau, ledit oligosaccharide étant le sucrose octasulfate.

Selon un premier aspect, l'invention concerne ainsi un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3 , ledit oligosaccharide étant le sucrose octasulfate.

Selon un deuxième aspect, l'invention concerne aussi une composition pharmaceutique comprenant un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, ledit oligosaccharide étant le sucrose octasulfate.

Selon un troisième aspect, l'invention concerne enfin un pansement comprenant un oligosaccharide polysulfaté synthétique ayant 1 à 4 unités oses, ses sels, ou ses complexes, en particulier un sel de potassium de sucrose octasulfate, pour son utilisation pour augmenter l'oxygénation de la peau lors du traitement des plaies ischémiques de grade 2 et/ou 3, ledit oligosaccharide étant le sucrose octasulfate.

### Description détaillée de l'invention

Oligosaccharides polysulfatés synthétiques ayant 1 à 4 unités oses

L'oligosaccharide selon l'invention est le sucrose.

On entend par « oligosaccharide polysulfaté » au sens de la présente invention un oligosaccharide dont tous les groupes hydroxyles de chaque ose ont été substitués par un groupe sulfate.

L'oligosaccharide polysulfaté utilisé dans le cadre de la présente invention est le sucrose octasulfate.

Les oligosaccharides polysulfatés utilisés dans le cadre de la présente invention peuvent se présenter sous forme de sels ou complexes.

A titre d'exemple de sels, on peut citer les sels de métal alcalin tels que les sels de sodium, de calcium ou de potassium ; les sels d'argent ; ou encore les sels d'acide aminé.

A titre d'exemple de complexes, on peut citer les complexes d'hydroxyaluminium.

Dans le cadre de la présente invention, des composés particulièrement préférés sont les suivants :
- le sel de potassium du sucrose octasulfate ;
- le sel d'argent du sucrose octasulfate ; et
- le complexe hydroxyaluminium du sucrose octasulfate, appelé communément sucralfate.

En particulier, dans le cadre de la présente invention, les oligosaccharides polysulfatés utilisés sont de préférence les sels de potassium plutôt que les sels d'aluminium du sucrose octasulfate.

Les oligosaccharides polysulfatés utilisés dans le cadre de la présente invention peuvent se présenter sous forme de poudre micronisée ou sous forme solubilisée.

Un exemple d'oligosaccharide polysulfaté utilisé dans le cadre de la présente invention est le sel de potassium du sucrose octasulfate (connu sous l'abréviation KSOS), commercialisé dans le produit Urgotul^{®} Start par les Laboratoires URGO.

Dans le cadre de la présente invention, les oligosaccharides polysulfatés utilisés peuvent être associés à un tampon phosphate salin (PBS).

### Composition

L'invention concerne aussi une composition pharmaceutique comprenant un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, ledit oligosaccharide étant le sucrose octasulfate.

Selon un mode de réalisation particulier, ladite composition pharmaceutique comprend un oligosaccharide polysulfaté synthétique ayant 1 à 4 unités oses, ses sels, ou ses complexes, à une concentration supérieure ou égale à 70 mg/mL, pour augmenter l'oxygénation de la peau lors du traitement des plaies ischémiques, de préférence 100 mg/mL, et plus préférentiellement comprise entre 100 et 1000 mg/mL, ledit oligosaccharide étant le sucrose octasulfate.

L'oligosaccharide polysulfaté synthétique utilisé dans la composition pharmaceutique selon l'invention est le sucrose octasulfate, plus particulièrement le sel de potassium de sucrose octasulfate.

### Substance active additionnelle

D'une façon générale, les composés oligosaccharides polysulfatés synthétiques selon l'invention pourront être utilisés seuls ou en mélange de deux ou plus d'entre eux, ou encore en combinaison avec une (ou plusieurs) autre(s) substance(s) active(s), par exemple dans des compositions telles que mentionnées ci-dessus.

De manière générale, les actifs sont choisis parmi les antibactériens, les antiseptiques, les anti-douleurs, les anti-inflammatoires, les actifs favorisant la cicatrisation, les agents dépigmentants, les antiprurigineux, les filtres UV, les agents apaisants, les agents hydratants, les agents antioxydants, et leurs mélanges.

De manière générale, les actifs sont choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques, des sels d'argent ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'ibuprofène, le Kétoprofène, le Flurbiprofène, le Diclofénac, l'Acéclofénac, le Kétorolac, le Méloxicam, le Piroxicam, le Ténoxicam, le Naproxène, l'indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de Centella Asiatica, la papaïne, les silicones, les huiles essentielles de thym, de niaouli, de romarin et de sauge, l'acide hyaluronique, l'Allantoïne, -Hema'tîte (gattefossé), Vitamine C, TEGO Pep 4-17( evonik), Toniskin (silab), Collageneer (Expanscience), Timecode (Seppic), Gatuline skin repair (gattefossé), Panthenol, PhytoCellTec Alp Rose (Mibelle Biochemistry), Erasyal(libragen), Serilesine (Lipotec), Heterosides de Talapetraka (beyer), Stoechiol(codif), macarose (Sensient), Dermaveil (Ichimaru Pharcos), Phycosaccaride AI (Codif) ;
- les agents dépigmentants tels que l'acide kojique (Kojic Acid SL^{®} - Quimasso (Sino Lion)), l'Arbutine (Olevatin^{®} - Quimasso (Sino Lion)), le mélange de palmitoylpropyl de sodium et d'extrait de nénuphar blanc (Sepicalm^{®} - Seppic), l'undécylénoyl phénylalanine (Sepiwhite^{®} - Seppic), les antiprurigineux : hydrocotisone, enoxolone, diphenyhydramine, antihistaminique à application locale anti H1 ;
- les actifs hydratants tels que xpermoist (lipotec), Acide hyaluronique, Urée, acides gras, Glycérine, Cires, Exossine( unipex) ;
- les filtres UV tels que Parsol MCX, Parsol 1789 ;
- les agents apaisants tels que de la camomille, du bisabolol, du xanthalène, de l'acide glycyrrhébénique, tanactine (CPN), Calmiskin (Silab) ;
- les agents anti-oxydants, tels que la vitamine E.

Selon un mode préféré de réalisation, les composés oligosaccharides selon l'invention peuvent être utilisés en combinaison avec un agent anti-oxydant.

### Galénique

Les oligosaccharides polysulfatés synthétiques utilisés dans le cadre de la présente invention peuvent être administrés par voie topique, et notamment mis en œuvre au sein d'une formulation galénique, sous la forme d'une composition, comme par exemple un gel, une solution, une émulsion, une crème, des granules, des capsules (de tailles variables allant du nano ou micromètre au millimètre), qui permettra leur application directement au niveau de la plaie. Alternativement, les composés utilisés dans le cadre de la présente invention peuvent être mis en oeuvre au sein d'une solution pour injection sous-cutanée.

S'ils sont employés en mélange de deux ou plusieurs d'entre eux ou encore en combinaison avec une ou plusieurs autres substances actives, ces composés pourront être incorporés dans la même formulation galénique ou dans des formulations galéniques distinctes.

Bien entendu, la quantité d'oligosaccharides polysulfatés synthétiques selon l'invention utilisée dans la formulation galénique est adaptée en fonction de la cinétique recherchée ainsi que des contraintes spécifiques liées à sa nature, solubilité, résistance à la chaleur, etc.

### Pansement

De manière préférentielle, les composés oligosaccharides polysulfatés synthétiques utilisés dans le cadre de la présente invention, ou une formulation galénique les contenant, seront intégrés à un pansement.

Par pansement, on entend désigner, au sens de la présente demande, tous types de pansements utilisés pour le traitement des plaies.

L'invention concerne ainsi un pansement comprenant un oligosaccharide polysulfaté synthétique ayant 1 à 4 unités oses, ses sels, ou ses complexes, en particulier un sel de potassium de sucrose octasulfate, pour augmenter l'oxygénation de la peau lors du traitement des plaies ischémiques de grade 2 et/ou 3, ledit oligosaccharide étant le sucrose octasulfate.

Le composé oligosaccharide polysulfaté synthétique selon l'invention, et notamment le sel de potassium de sucrose octasulfate ou une formulation galénique le contenant, pourra être incorporé dans un élément quelconque de la structure d'un pansement sous réserve que ce composé puisse entrer directement ou indirectement en contact avec la surface de la plaie.

De préférence et afin de favoriser une action rapide, ce composé (ou une formulation galénique le contenant) peut être incorporé dans la couche du pansement qui vient en contact avec la plaie ou déposé sur la surface du pansement qui vient en contact avec la plaie.

De telles techniques de dépôt sont bien connues de l'homme de l'art et certaines sont par exemple décrites dans la demande de brevet WO 2006/007814.

Avantageusement, le sel de potassium du sucrose octasulfate (ou une formulation galénique le contenant) pourra ainsi être déposé, de façon continue ou discontinue, sur la surface destinée à venir au contact de la plaie :
- soit sous forme liquide, par exemple par vaporisation d'une solution ou suspension le contenant ;
- soit sous forme solide, par exemple par tamisage d'une poudre le contenant.

La couche ou surface venant en contact avec la plaie pourra être constituée par exemple d'un matériau absorbant telle qu'une mousse absorbante hydrophile en polyuréthane ; un matériau textile telle qu'une compresse, comme par exemple un non tissé, un film, un voile de fibres ; un matériau adhésif absorbant ou non ; une structure interface adhérente ou non.

Typiquement, un pansement comprend au moins une couche ou matrice, adhésive ou non.

De façon alternative, la couche ou surface venant en contact avec la plaie pourra être constituée par exemple d'une trame textile, de préférence en polyester telle que décrite dans la demande de brevet WO 01/70285 ou dans la demande de brevet WO2013/093298 sur laquelle sera enrobée, ou enduite, une matrice élastomérique comprenant un oligosaccharide polysulfaté synthétique ayant 1 à 4 unités oses, ses sels, ou ses complexes, en particulier un sel de potassium de sucrose octasulfate, telle que décrite dans la demande de brevet WO2008/149035 ou dans la demande WO2014/009488.

Les composés oligosaccharides polysulfatés synthétiques selon l'invention, ou une formulation galénique les contenant, peuvent être incorporés dans un élément quelconque de la structure d'un pansement, par exemple dans la matrice.

L'invention a ainsi pour objet un pansement comprenant une trame textile enduite d'une matrice élastométique comprenant un oligosaccharide polysulfaté synthétique ayant 1 à 4 unités oses, ses sels, ou ses complexes, en particulier un sel de potassium de sucrose octasulfate, pour son utilisation pour augmenter l'oxygénation de la peau lors du traitement des plaies ischémiques de grade 2 et/ou 3, ledit oligosaccharide étant le sucrose octasulfate.

Selon un mode de réalisation préféré, l'invention concerne ainsi un pansement comprenant un oligosaccharide polysulfaté synthétique ayant 1 à 4 unités oses, ses sels, ou ses complexes, en particulier un sel de potassium de sucrose octasulfate pour augmenter l'oxygénation de la peau lors du traitement des plaies ischémiques de grade 2 et/ou 3, ledit oligosaccharide étant le sucrose octasulfate, ledit pansement comprenant une trame textile enduite d'une matrice élastométique, et ladite matrice comprenant ledit oligosaccharide polysulfaté synthétique.

De façon générale, on pourra jouer sur la galénique ou la structure du pansement pour obtenir un profil de relargage du sel de potassium de sucrose octasulfate spécifique, rapide ou retardé, selon les besoins.

Bien entendu, la quantité de sel de potassium de sucrose octasulfate utilisée dans la formulation galénique ou dans le pansement sera adaptée en fonction de la cinétique recherchée ainsi que des contraintes spécifiques liées à sa nature, solubilité, résistance à la chaleur, etc.

Selon une variante de l'invention, le composé oligosaccharide polysulfaté synthétique selon l'invention peut être incorporé dans un pansement absorbant à base de fibres gélifiantes, comme par exemple le produit AQUACEL^{®} commercialisé par la société CONVATEC.

Très souvent, lors de la pose de ces pansements, le personnel soignant maintient ces derniers en place à l'aide d'une bande ou recouvre ces derniers d'un élément secondaire tel qu'un second pansement absorbant ou une bande de contention. Il est donc utile que le pansement reste fixé sur la plaie afin que le personnel soignant conserve les mains libres pour positionner ces éléments secondaires. D'une façon générale, tout type d'adhésif couramment employé dans les pansements pourra être utilisé à cet effet.

Afin de ne pas altérer les tissus sains ou les berges de la plaie, notamment lors du retrait du pansement, on préfèrera un adhésif ayant la propriété d'adhérer à la peau sans adhérer à la plaie.

A titre d'exemple d'un tel adhésif, on peut ainsi citer les adhésifs à base d'élastomères de silicone ou de polyuréthane, tels que les gels de silicone ou de polyuréthane, et les adhésifs hydrocolloïdes.

De tels adhésifs hydrocolloïdes sont notamment constitués d'une matrice élastomérique à base d'un ou plusieurs élastomères choisis parmi les polymères séquencés poly(styrène-oléfine-styrène) en association avec un ou plusieurs composés choisis parmi les plastifiants, tels que les huiles minérales, des résines tackifiantes et, si nécessaire, des antioxydants, dans laquelle est incorporée une quantité, de préférence faible, d'hydrocolloïdes (de 3 à 20% en poids) comme par exemple la carboxyméthylcellulose de sodium ou des polymères superabsorbants comme les produits commercialisés sous la dénomination LUQUASORB^{®} par la société BASF.

Selon un mode préféré de réalisation, les composés oligosaccharides polysulfatés synthétiques utilisés dans le cadre de la présente invention, ou une formulation galénique les contenant, seront intégrés à un pansement comprenant un adhésif hydrocolloïde, ledit oligosaccharide polysulfaté étant incorporé dans ledit adhésif de préférence en une quantité comprise entre 1 et 15% en poids, de préférence encore entre 5 et 10% en poids, par rapport au poids de l'adhésif.

La formulation de tels adhésifs hydrocolloïdes est bien connue de l'homme de l'art et décrite par exemple dans les demandes de brevet FR2783412, FR2392076 et FR2495473.

L'utilisation d'un filet d'adhésif sur le non tissé permet d'une façon particulièrement avantageuse de diminuer ou d'éviter le risque que de petites fibrilles du matériau textile viennent au contact de la plaie et s'accrochent aux tissus, en provoquant ainsi une sensation douloureuse au retrait, voire un obstacle au processus de cicatrisation de la plaie.

Selon une variante de réalisation préférée de la présente invention, le composé oligosaccharide polysulfaté synthétique selon l'invention est incorporé dans un tel adhésif à une concentration compatible avec sa solubilité et sa résistance à la chaleur.

Sur la base de ces critères, le composé oligosaccharide polysulfaté synthétique selon l'invention est utilisé de préférence en une quantité comprise entre 1 et 15% en poids, et de préférence encore entre 5 et 10% en poids, par rapport au poids total de l'adhésif.

Si l'on souhaite augmenter l'absorption de ce pansement non tissé, on pourra associer ce dernier avec une couche absorbante additionnelle, et de préférence une couche absorbante qui ne gélifie pas, comme en particulier une compresse telle que celle utilisée dans le produit URGOTUL^{®} Duo ou URGOTUL^{®} Trio, une mousse hydrophile absorbante, de préférence une mousse polyuréthane hydrophile présentant une capacité d'absorption supérieure à celle du non tissé telle que celle utilisée dans le produit CELLOSORB^{®}.

Selon un mode préféré de réalisation, le composé oligosaccharide polysulfaté synthétique selon l'invention est incorporé dans un pansement non tissé, associé avec une couche absorbante additionnelle, et de préférence une couche absorbante qui ne gélifie pas, comme en particulier une compresse, ledit oligosaccharide étant le sucrose octasulfate.

Selon un autre mode préféré de réalisation, le composé oligosaccharide polysulfaté synthétique selon l'invention est incorporé dans un pansement non tissé, associé avec une couche absorbante additionnelle, et de préférence une couche absorbante qui ne gélifie pas, comme en particulier une mousse hydrophile absorbante, de préférence une mousse polyuréthane hydrophile présentant une capacité d'absorption supérieure à celle du non tissé, ledit oligosaccharide étant le sucrose octasulfate.

Le non tissé et la mousse peuvent être associés par des techniques bien connues de l'homme de l'art, par exemple par calandrage à chaud à l'aide d'une poudre thermofusible à base de polymères TPU/polycaprolactone.

Cette technique est couramment employée pour le liage entre eux de non tissés destinés au marché médical.

Enfin, cette mousse ou le non tissé (lorsque celui-ci est utilisé seul) peuvent être recouverts d'un support pour protéger la plaie de l'extérieur.

Ce support peut être de taille supérieure à celle des autres couches et rendu adhésif de façon continue ou discontinue sur sa face venant en contact avec la plaie afin d'optimiser le maintien du pansement lors de son usage, en particulier si la plaie se situe sur des zones corporelles non planes.

Ce support et son adhésif sont de préférence imperméables aux fluides mais très perméables à la vapeur d'eau afin de permettre une gestion optimale des exsudats absorbés par le pansement et éviter les problèmes de macération.

De tels supports sont bien connus de l'homme du métier et sont constitués par exemple de films respirants et imperméables tels que des films de polyuréthane, des complexes mousse/film ou non tissé/film.

### Additifs

Outre les agents actifs, les composés oligosaccharides polysulfatés synthétiques selon l'invention pourront être utilisés en combinaison avec un (ou plusieurs) additifs couramment utilisés dans la préparation des pansements. Ces additifs peuvent notamment être choisis parmi les parfums, les conservateurs, les vitamines, la glycérine, l'acide citrique, etc.

### Application thérapeutique

Le composé oligosaccharide polysulfaté synthétique selon l'invention est utilisé pour augmenter l'oxygénation de la peau lors du traitement des plaies ischémiques de grade 2 et/ou 3.

Selon un mode de réalisation, l'invention concerne un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, ledit oligosaccharide étant le sucrose octasulfate.

De préférence, l'oligosaccharide pour son utilisation selon l'invention est utilisé à une concentration supérieure ou égale à 70 mg/mL, de préférence 100 mg/mL, et plus préférentiellement comprise entre 100 et 1000 mg/mL.

Selon l'invention, on entend par « augmenter l'oxygénation de la peau », le fait d'augmenter localement, directement au niveau de la plaie, la concentration en oxygène, et ceci dans le but de favoriser la cicatrisation.

L'oxygénation de la peau est déterminée par la mesure de la TcPO₂ (c'est-à-dire la pression transcutanée en oxygène, en d'autres termes la pression de l'oxygène qui diffuse à travers la peau au niveau de la plaie). Dans le terme « TcPO₂ », Tc se réfère au terme « transcutané », P à la « Pression » et O₂ à l'oxygène.

La TcPO₂ est de préférence mesurée à l'aide d'un appareil TCM400 (Radiometer), en plaçant les électrodes au contact de la plaie, tel que décrit dans les exemples ou dans la publication Izzo V, et al., Rearfoot Transcutaneous Oximetry is a Useful Tool to Highlight Ischemia of the Heel. Cardiovasc Intervent Radiol. 2017;40(1):120-4.

Selon l'invention, la TcPO2 est de préférence mesurée au premier jour du traitement, puis mensuellement jusqu'à la cicatrisation de la plaie.

Selon un mode de réalisation, l'oligosaccharide pour son utilisation selon l'invention permet une augmentation de la TcPO₂ d'au moins 10 mm Hg entre le premier jour du traitement et la cicatrisation de la plaie, plus particulièrement le traitement des plaies ischémiques de grade 2 et/ou 3, ledit oligosaccharide étant le sucrose octasulfate.

Selon l'invention, on entend par « plaies ischémiques », les plaies résultant d'une diminution de l'apport sanguin artériel. Les plaies ischémiques sont classées en 3 catégories : les plaies présentant une ischémie faible (grade 1), les plaies présentant une ischémie modérée (grade 2), et enfin les plaies présentant une ischémie critique (grade 3). La distinction entre ces différents grades est établie par la classification ci-après, décrite dans la publication « The Society for Vascular Surgery Lower Extremity Threatened Limb Classification System: Risk stratification based on Wound, Ischemia, and foot Infection (WIfI) », Joseph L.Mills Sr.MD, Michael S.Conte MD et al. , Journal of Vascular Surgery.

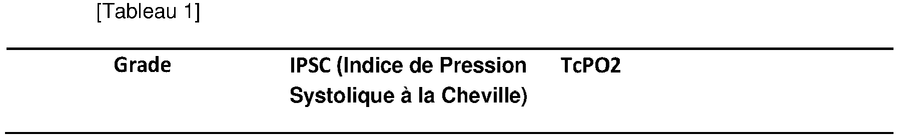

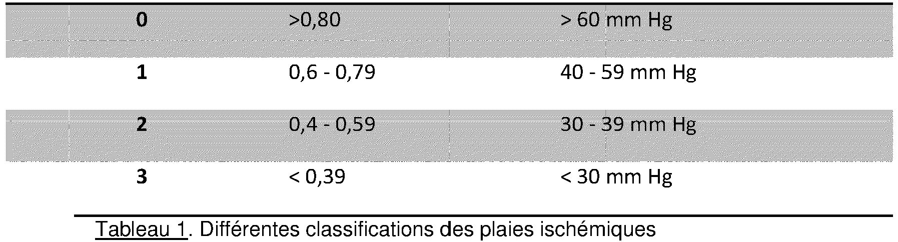

Selon l'invention, on entend par « ischémie » un défaut d'apport sanguin artériel vers un tissu (par exemple la plaie), et un défaut d'oxygénation dudit tissu.

La présente invention vise les plaies ischémiques de grade 2 et/ou 3. La présente invention vise ici à cibler les plaies présentant une ischémie modérée ou critique, afin de traiter la plaie, traiter l'ischémie de ladite plaie ou diminuer l'ischémie de ladite plaie. En d'autres termes, selon ce mode de réalisation, la présente invention vise les plaies ischémiques présentant une valeur de TcPO₂ inférieure à 30 mm Hg, jusqu'à une valeur inférieure ou égale 39 mm Hg.

Selon un mode de réalisation, les « plaies ischémiques de grade 2 et/ou 3 » s'entend des plaies ischémiques de grade 2 et/ou des plaies ischémiques de grade 3 et/ou des plaies ischémiques de grade 2 et 3. Selon un mode de réalisation préféré, il s'agit des plaies ischémiques de grade 2 et 3.

Selon l'invention, la diminution de l'ischémie d'une plaie s'entend plus particulièrement de l'évolution de la plaie ischémique de grade 3 vers le grade 2, et/ou l'évolution de la plaie ischémique de grade 2 vers le grade 1. La présente invention consiste ici en un traitement des plaies à un stade avancé, voire très avancé, et notamment en ciblant l'ischémie. La diminution de l'ischémie permet en effet de favoriser l'évolution de la plaie vers la cicatrisation.

Selon l'invention, les plaies ischémiques sont choisies parmi l'ulcère de jambe artériel, l'ulcère de jambe mixte, l'escarre de décubitus, et l'ulcère du pied du diabétique neuro-ischémique. Selon un mode de réalisation particulier, les plaies ischémiques sont choisies parmi l'ulcère de jambe artériel, l'ulcère de jambe mixte, et l'escarre de décubitus. L'ulcère du pied diabétique (UPD ou DFU en anglais) est défini par « une plaie profonde localisée sous la cheville chez un patient diabétique, indépendamment de sa durée » (FID, 2005). En effet, la cause première de l'absence de cicatrisation de ces plaies diabétiques est liée à une biodisponibilité du glucose exacerbée. Celle-ci induit de nombreuses modifications physiologiques et métaboliques, telles qu'un épaississement de la peau, un stress oxydatif important pouvant conduire à une neuropathie ou à une ischémie pouvant être faible, modérée à critique. L'ischémie est donc un facteur de risque majeur de retard de la cicatrisation de l'ulcère du pied diabétique.

Selon un mode de réalisation particulier, les plaies ischémiques sont choisies parmi l'ulcère de jambe artériel ischémique de grade 2 et/ou 3, l'ulcère de jambe mixte ischémique de grade 2 et/ou 3, l'escarre de décubitus ischémique de grade 2 et/ou 3, et l'ulcère du pied du diabétique neuro-ischémique ischémique de grade 2 et/ou 3. Selon un mode de réalisation encore plus particulier, les plaies ischémiques sont choisies parmi l'ulcère de jambe artériel ischémique de grade 2 et/ou 3, l'ulcère de jambe mixte ischémique de grade 2 et/ou 3, et l'escarre de décubitus ischémique de grade 2 et/ou 3.

Selon un mode particulier de réalisation, l'ulcère du pied diabétique traité dans le cadre de la présente demande présente une dimension inférieure à 5 cm², c'est-à-dire que la plaie s'inscrit dans un cercle dont l'aire est de 5 cm².

Selon un mode encore préféré de réalisation, l'ulcère du pied diabétique traité dans le cadre de la présente demande n'est pas récalcitrant, c'est-à-dire qu'il s'est formé depuis moins de 6 mois.

Selon un mode particulier de réalisation, l'ulcère du pied diabétique neuro-ischémique objet de la présente demande présente :
- un Indice de Pression Systolique à la Cheville (IPSC) inférieur ou égal à 0.9,
   ou
- un Indice de Pression Systolique à la Cheville (IPSC) supérieur à 0.9 associé à un Indice de Pression Systolique à l'Orteil (IPSO), inférieur ou égal à 0.7.

L'ulcère de jambe artériel est défini par « une plaie chronique de la jambe d'origine vasculaire ne cicatrisant pas depuis plus d'un mois ». Cette pathologie est caractérisée par une perte de substance cutanée de l'épiderme et du derme, directement due à une ischémie par défaut de perfusion artérielle du membre.

Selon un mode particulier de réalisation, l'ulcère de jambe artériel présente un Indice de Pression Systolique à la Cheville (IPSC) compris entre 0.6 et 0.8.

L'ulcère de jambe mixte est défini par « une plaie chronique de la jambe d'origine veineuse et vasculaire, ne cicatrisant pas depuis plus d'un mois ». Cette pathologie est caractérisée par une perte de substance cutanée de l'épiderme et du derme, directement due à une ischémie par défaut de perfusion veineuse et artérielle du membre.

Selon un mode particulier de réalisation, l'ulcère de jambe artériel présente un Indice de Pression Systolique à la Cheville (IPSC) compris entre 0.6 et 0.8.

L'escarre de décubitus est définie par « une plaie chronique provoquée par une pression constante ». Elle touche aussi bien l'épiderme, le derme que les os. Les zones cutanées particulièrement sensibles sont celles qui sont situées directement à la surface des os. Cette pathologie est caractérisée par une nécrose ischémique. Les escarres sont classées en quatre stades de gravité croissante :
- stade 1 : rougeur permanente et parfois sensation de chaleur au niveau de la zone de peau concernée, la peau est intacte.
- stade 2 : formation de vésicules cutanées, éraflure de l'épiderme et parfois plaie superficielle.
- stade 3 : l'épiderme, le derme ainsi que le tissu conjonctif sont touchés. Une plaie profonde apparaît.
- stade 4 : plaie profonde s'étendant jusqu'au tissu musculaire et osseux.

L'activité des oligosaccharides polysulfatés synthétiques selon l'invention a été mise en évidence dans les exemples non limitatifs suivants.

### Exemples

### Exemple 1 : Augmentation de l'oxygénation de la peau lors du traitement de l'ulcère du pied du diabétique neuro-ischémique

Le but de cette étude a été d'évaluer l'effet des valeurs de TcPO₂ (mm Hg) dans l'utilisation d'un pansement à base de sucrose octasulfate au cours du processus de cicatrisation des ulcères de pied du diabétique neuro-ischémique.

### 1. Matériel & Méthodes

### Patients

L'étude a été menée sur 11 patients. Les critères d'inclusion des patients dans l'étude étaient les suivants :
- patients âgés de plus de 18 ans diagnostiqués avec un diabète *mellitus* de type 1 ou 2, présentant un ulcère du pied diabétique neuro-ischémique non infecté de grade IC ou IIC, tel que défini par le système de classification des plaies diabétiques de l'Université du Texas (Armstrong DG, Lavery LA, Harkless LB. Validation of a diabetic wound classification system. The contribution of depth, infection, and ischemia to risk of amputation. Diabetes Care. 1998;21(5):855-9), un contrôle glycémique confirmé par un taux d'HbA_{1c} ≤ 10% (85.8 mmol/mol) au cours des trois mois précédents. Ces patients étaient candidats pour commencer un traitement avec un pansement à base de sucrose octasulfate, sur une surface de plaie comprise entre 1 et 30 cm².

Les patients souffrant d'ischémie d'un membre critique, de maladie rénale ou de dialyse en phase terminale, de présence d'un œdème dû à une maladie vasculaire, rénale ou cardiaque, les patients atteints de bronchopneumopathie chronique obstructive (BPCO) pouvant altérer la saturation en oxygène au niveau systémique, les patients ayant subi un accident vasculaire cérébral au cours des trois derniers mois, ayant un pied de Charcot aigu et ceux ayant subi une revascularisation chirurgicale au cours des trois mois précédant leur entrée dans l'étude, ont été exclus.

### Évaluation des patients

La neuropathie a été confirmée à l'aide d'un monofilament Semmes-Weinstein de 10 g et/ou d'un biothésiomètre (Me.Te.Da. s.r.l., Via Silvio Pellico, 4, 63074 San Benedetto del Tronto, Italie) (Armstrong DG, Lavery LA, Harkless LB. Validation of a diabetic wound classification system. The contribution of depth, infection, and ischemia to risk of amputation. Diabetes Care. 1998;21(5):855-9, ou Schaper NC, van Netten JJ, Apelqvist J, Bus SA, Hinchliffe RJ, Lipsky BA. Practical Guidelines on the prevention and management of diabetic foot disease (IWGDF 2019 update). Diabetes Metab Res Rev. 2020;36 Suppl 1:e3266).

Les patients neuro-ischémiques étaient définis comme suit : ayant un Indice de Pression Systolique à la Cheville (IPSC) inférieur ou égal à 0.9 ; ou un Indice de Pression Systolique à la Cheville (IPSC) supérieur à 0.9 associé à un Indice de Pression Systolique à l'Orteil (IPSO), inférieur ou égal à 0.7.

Les valeurs de TcPO₂ ont été mesurées à l'aide de l'appareil de mesure TCM400 (Radiometer, Copenhague, Danemark), en plaçant l'électrode sur l'angiosome de l'artère tibiale postérieure ou de *dorsalis pedis* en fonction de la localisation de l'ulcère (Izzo *et al.*)*.* Les patients ont été placés en position couchée pendant les 10 minutes d'examen et on leur a demandé de ne pas bouger ni parler. Après calibrage, l'électrode a été placée au niveau de l'artère dorsale pédieuse dans tous les ulcères de pied de diabétique situés au niveau des orteils ou de l'avant-pied et au niveau de l'artère tibiale postérieure chez les patients qui avaient des ulcères de pied de diabétique au milieu ou à l'arrière-pied. Les valeurs de TcPO₂ ont été évaluées au jour 0, sur une base mensuelle et le jour de la fermeture/cicatrisation de la plaie.

### Évaluation de la plaie

La cicatrisation des plaies a été évaluée sur la base des améliorations du système de notation de Wollina (Wollina U, et al., Some effects of a topical collagen-based matrix on the microcirculation and wound healing in patients with chronic venous leg ulcers: preliminary observations. Int J Low Extrem Wounds. 2005;4(4):214-24), et de la surface de la zone de la plaie au jour 0 et tous les mois jusqu'à 20 semaines de suivi ou de cicatrisation. La cicatrisation a été définie comme une épithélialisation complète sans aucun drainage, confirmée au moins 10 jours après que la fermeture de la plaie ait été notée pour la première fois (Edmonds M, et al., Sucrose octasulfate dressing versus control dressing in patients with neuroischaemic diabetic foot ulcers (Explorer): an international, multicentre, double-blind, randomised, controlled trial. Lancet Diabetes Endocrinol. 2018;6(3):186-96)).

Tous les patients ont reçu des soins standards consistant à débrider l'ulcère et à le décharger correctement en suivant les recommandations des directives de l'IWGDF (Bus SA, et al., Guidelines on offloading foot ulcers in persons with diabetes (IWGDF 2019 update). Diabetes Metab Res Rev. 2020;36 Suppl 1:e3274.). Les ulcères de pied de diabétique étaient habillés d'un pansement en sucrose octasulfate (UrgoStart Contact, 10 × 10 cm, Laboratoires Urgo Medical, Chenôve, France). Il s'agit d'un pansement non adhérent, non occlusif, avec une couche de contact souple composée d'une maille de polyester imprégnée d'une matrice lipidocolloïde contenant du sel de potassium de sucrose octasulfate (NOSF : facteur nano-oligosaccharide). Un prestataire de soins de santé senior a suivi tous les patients pour leurs soins dans le service en appliquant le pansement deux fois par semaine jusqu'à la fin de l'étude. En outre, le même clinicien a effectué le suivi sur une base mensuelle pour enregistrer les variables de l'étude.

### Analyse statistique

L'hypothèse de normalité de toutes les variables continues a été vérifiée à l'aide du test de Shapiro-Wilk. Les variables distribuées normalement (test de Shapiro-Wilk avec p > 0,05) ont été rapportées comme moyenne et écart-type.

Le test t de Student pour les échantillons appariés a été utilisé pour explorer les différences dans les valeurs de TcPO₂, le score de Wollina et la surface de la plaie au sein du traitement avec le pansement à l'octasulfate en raison de la distribution normale des variables.

Toutes les analyses statistiques ont été effectuées en utilisant la version 25.0 des statistiques SPSS pour Mac OS (SPSS, Chicago, IL, USA). Les valeurs p <0,05 ont été considérées comme statistiquement significatives, avec des intervalles de confiance de 95%.

### 2. Résultats

Au total, 11 patients ont été inclus dans cette étude pilote et suivis - jusqu'à la guérison de l'ulcère. Les caractéristiques démographiques, le diabète sucré et les complications du pied au départ sont présentés dans le Tableau 2.

### [Tableau 2]

**Tableau 2. Caractéristiques des 11 patients de l'étude (Abréviations: SD, déviation standard; TcPO2, Pression transcutanée en oxygène)**

| **Caractéristiques** | **Patients (N=11)** |
|---|---|
| Homme, n (%) | 8 (72.7 %) |
| Femme, n (%) | 3 (27.3 %) |
| Age moyen ± SD (années) | 61.91 +/- 8.87 |
| Indice de masse corporelle (kg/cm²), moyen ± SD | 30.05 +/- 4.98 |
| Diabète *mellitus* de type 1, n (%) | 3 (27.3 %) |
| Diabète *mellitus* de type 2, n (%) | 8 (72.7 %) |
| Hémoglobine glyquée (%), moyenne ± SD | 7.62 +/- 1.15 |
| Durée du diabète *mellitus* (année), moyenne ± SD | 26.36 +/- 13.60 |

| **Complications du diabète *mellitus*** | |
|---|---|
| Rétinopathie, n (%) | 5 (54.5 %) |
| Néphropathie, n (%) | 2 (18.2 %) |
| Cardiopathie, n (%) | 2 (18.2 %) |
| Hypertension, n (%) | 8 (72.7 %) |

| **Traitements systémiques actuels** | |
|---|---|
| Antihypertenseur, n (%) | 8 (72.7%) |
| Hypolipidémiant, n (%) | 10 (90.9%) |
| Antiplaquettaire, n (%) | 9 (81.8%) |
| Fumeur, n (%) | 4 (36.4 %) |
| Antécédent d'ulcère du pied du diabétique, n (%) | 9 (81.8 %) |
| Amputation mineure précédente, n (%) | 7(63.6 %) |
| Présence d'un pouls dorsal du pied, n (%) | 2 (18.2 %) |
| Présence d'un pouls de l'artère tibiale postérieure, n (%) | 3 (27.3 %) |
| Index de pression tibio-brachial, moyen ± SD | 1.19 +/- 0.28 |
| Index de pression brachial à l'orteil, moyen ± SD | 0.60 +/- 0.11 |
| Pression systolique à l'orteil (mm Hg), moyenne ± SD | 72.62 +/- 9.60 |
| TcPO₂ Jour 0 (mm Hg), moyenne ± SD | 33.54 +/- 11.16 |

Quatre ulcères du pied du diabétique (36,4%) étaient situés dans l'hallux, deux (18,2%) sous la première tête métatarsienne (MTH), un (9,1%) sous la deuxième MTH, un (9,1%) sous la troisième MTH, un (9,1%) sous le cinquième MTH, un (9,1%) au milieu du pied et enfin, et un (9,1%) au talon. Les caractéristiques des plaies sont présentées dans le Tableau 3.

### [Tableau 3]

**Tableau 3. Caractéristiques des plaies (Abréviations : SD, déviation standard; IQR, écart interquartile)**

| **Caractéristiques des plaies** | **Patients (N=11)** |
|---|---|
| Durée de la plaie (semaine), médian [IQR] | 8 [48 - 2] |
| Surface de la plaie (cm²), médian [IQR] | 1.30 [1.60 - 1] |
| Score de Wollina moyen ± SD | 4.18 +/- 1.72 |

| **Classification du Texas,** | |
|---|---|
| IC: Ischémie mais pas d'infection, plaie superficielle, n (%) | 6 (54.5%) |
| IIC: Ischémie mais pas d'infection, plaie pénétrant les tendons, la capsule, n (%) | 5 (45.5%) |

| **Etat de la peau périlésionnelle** | |
|---|---|
| Hyperkératose, n (%) | 6 (54.5%) |
| Macéré, n (%) | 3 (27.3%) |
| Décollement, n (%) | 2 (18.2%) |

| **Quantité d'exsudat** | |
|---|---|
| Faible, n (%) | 4 (36.4%) |
| Moyenne, n (%) | 5 (45.5%) |
| Elevée, n (%) | 2 (18.2%) |

| **Tissu de lit de la plaie** | |
|---|---|
| Tissu de granulation, n (%) | 7 (63.6%) |
| Tissu mou, n (%) | 2 (18.2%) |
| Mixte (granulation et mou), n (%) | 2 (18.2%) |

La fréquence du débridement tissulaire a été considérée comme nécessaire chez chaque patient. Les patients ont reçu un traitement d'un dispositif de décharge en fonction de l'emplacement de l'ulcère (Tableau 4).

### [Tableau 4]

**Tableau 4. Interventions sur la plaie durant la période de suivi**

| **Interventions sur la plaie** | **Patients (N=11)** |
|---|---|
| **Dispositif de décharge décrit** | |
| Dispositif de décharge à hauteur des genoux non amovible, n (%) | 5 (45.5%) |
| Dispositif de chaussure post-chirurgical, n (%) | 5 (45.5%) |
| Dispositif de soulagement du talon, n (%) | 1(9.1%) |

| **Débridement des plaies** | |
|---|---|
| Mécanique, n (%) | 3 (27.3%) |
| Chirurgie, n (%) | 8 (72.7%) |

| **TcPO₂** - **lieu de l'électrode** | |
|---|---|
| *Dorsalis pedis, n* (%) | 9 (81.8%) |
| Tibial postérieur, n (%) | 2 (18.2%) |

| **Pansement secondaire utilisé** | |
|---|---|
| Gaze, n (%) | 6 (54.5%) |
| Mousse, n (%) | 5 (45.5%) |

Tous les patients inclus dans l'étude ont guéri dans un délai médian de 8 semaines IQR [8 - 5].

Les valeurs de TcPO₂ après un traitement local par pansement contenant du sucrose octasulfate ont montré une augmentation (statistiquement significative) entre le jour 0 (33,54 +/- 11,16 mm Hg) et le jour de la fermeture de la plaie (45,27 +/- 13,62 mm Hg), valeur p <0,016 (Tableau 5).

### [Tableau 5]

**Tableau 5. Différences dans les valeurs de TcPO₂ au niveau du pied des patients après application d'un pansement contenant du sucrose octasulfate (N = 11) (Toutes les comparaisons ont été faites par rapport aux valeurs de TcPO₂ au jour 0. P < 0.05 indique une différence statistique significative)**

| Durée | Patients (N=11) | P - valeur |
|---|---|---|
| Jour 0 | 33.54 +/- 11.16 | - |
| Semaine 4 | 38.09 +/- 13.53 | .303 |
| Semaine 8 | 43.50 +/- 13.65 | .056 |
| Fermeture de la plaie | 45.27 +/- 13.62 | **.016** |

Pendant la période de suivi, les scores des plaies de Wollina ont montré une amélioration significative, passant d'un score moyen de 4,2 +/- 1,7 points au jour 0 à 5,4 +/- 1,3 points à la fin de l'étude (N = 11, p = 0,004).

Enfin, la surface médiane de la plaie au jour 0 était de 1,30 IQR [1,60 - 1] cm² et de 0,5 IQR [1,1 - 0,1] cm² à la semaine 4, avec une réduction significative de la surface de la plaie pendant la période de suivi (N = 11, p <. 001).

Ces résultats montrent donc qu'un oligosaccharide polysulfaté synthétique ayant 1 à 4 unités oses, ses sels, ou ses complexes, (plus particulièrement le sucrose octasulfate) permet d'augmenter l'oxygénation de la peau lors du traitement des plaies ischémiques.

### Exemple 2 : Augmentation de l'oxygénation de la peau lors du traitement de l'ulcère du pied du diabétique neuro-ischémique

L'étude de l'Exemple 1 a été complétée, et les résultats sont présentés dans le Tableau 6.

### [Tableau 6]

**Tableau 6. Différences dans les valeurs de TcPO₂ au niveau du pied des patients après application d'un pansement contenant du sucrose octasulfate (N = 34) (Toutes les comparaisons ont été faites par rapport aux valeurs de TcPO₂ au jour 0. P < 0.05 indique une différence statistique significative)**

| Durée | Patients (N=34) | P - valeur |
|---|---|---|
| Jour 0 | 31.29 ± 9.61 | - |
| Semaine 4 | 37.55 ± 12.96 | **0.004** |
| Semaine 8 | 37.26 ± 14.98 | **.04** |
| Semaine 12 | 46.14± 9.33 | **0.04** |
| Fermeture de la plaie | 42.87 ± 12.16 | **.007** |

Pendant la période de suivi, les scores des plaies de Wollina ont montré une amélioration significative, passant d'un score moyen de 3,95 +/- 1,94 points au jour 0 à 5,75 +/- 0,95 points à la fin de l'étude (p = 0,003).

Enfin, la surface médiane de la plaie au jour 0 était de 1,40 IQR [1,70 - 1,2] cm² et de 0,2 IQR [1,1 - 0,1] cm² à la semaine 4, avec une réduction significative de la surface de la plaie pendant la période de suivi (p <. 001).

### Exemple 3 : Evolution de l'oxygénation de la peau selon le grade de la plaie ischémique

Les 34 patients de l'Exemple 2 ont été séparés selon la classification de la plaie ischémique, telle que présentée dans le Tableau 1. L'évolution des valeurs de TcPO₂ au niveau du pied des patients après application d'un pansement contenant du sucrose octasulfate a été mesurée. L'analyse statistique a été réalisée comme indiquée dans l'Exemple 1.

Les résultats sont présentés dans le Tableau 7.

### [Tableau 7]

**Tableau 7. Différences dans les valeurs de TcPO₂ selon la classification de la plaie ischémique.**

| Classification de la plaie ischémique | TcPO₂ (Jour 0) | TcPO₂ (cicatrisation) | ΔTcPO₂ | P - valeur |
|---|---|---|---|---|
| 0 | - | - | - | - |
| 1 | 45,33± 4,27 | 51,22 ± 8,46 | 5,89 | 0.133 |
| 2 | 32,81 ± 2,27 | 44,27 ± 10,52 | 11,46 | 0.005 |
| 3 | 23,14 ± 3,05 | 36,57 ± 11,96 | 13,43 | 0.001 |

Les résultats montrent qu'au jour de la cicatrisation les plaies ischémiques de grade 3 (TcPO₂ < 30 mm Hg) ont évolué en plaies ischémiques de grade 2 (TcPO₂ < 30-39 mm Hg).

Les résultats montrent également que les plaies ischémiques de grade 2 (TcPO₂ < 30-39 mm Hg) ont évolué en plaies ischémiques de grade 1 (TcPO₂ < 40-59 mm Hg).

Au contraire, les plaies ischémiques de grade 1 restent classifiées comme des plaies ischémiques de grade 1 (TcPO₂ < 40-59 mm Hg).

Ces résultats montrent donc l'intérêt particulier conférée par l'invention pour le traitement des plaies ischémiques de grade 2 et/ou 3. L'oxygénation des plaies ischémiques de grade 2 et 3 est en effet améliorée de plus de 10 mm Hg entre le premier jour du traitement et la cicatrisation, ce qui est plus du double par comparaison avec l'augmentation de l'oxygénation des plaies ischémiques de grade 1.

## Revendications

1. Oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, ledit oligosaccharide étant le sucrose octasulfate et lesdits grades étant définis dans le Tableau 1 :

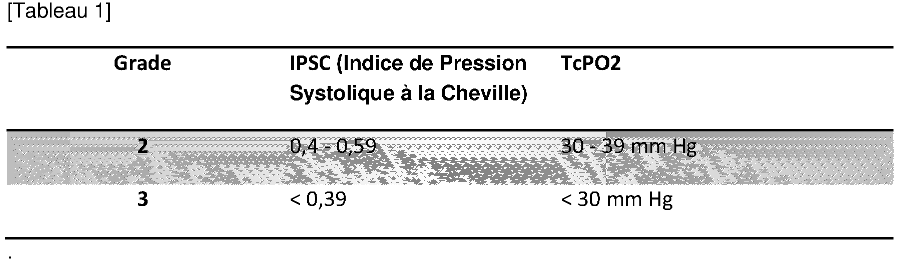

2. Oligosaccharide pour son utilisation selon la revendication 1, **caractérisé en ce que** les plaies ischémiques sont choisies parmi l'ulcère de jambe artériel, l'ulcère de jambe mixte, l'escarre de décubitus, et l'ulcère du pied du diabétique neuro-ischémique.

3. Oligosaccharide pour son utilisation selon l'une quelconque des revendications précédentes, **caractérisé en ce que** les plaies ischémiques sont choisies parmi l'ulcère de jambe artériel, l'ulcère de jambe mixte, et l'escarre de décubitus.

4. Oligosaccharide pour son utilisation selon l'une quelconque des revendications précédentes, **caractérisé en ce que** sa concentration est supérieure ou égale à 70 mg/mL, de préférence 100 mg/mL, et plus préférentiellement comprise entre 100 et 1000 mg/mL.

5. Oligosaccharide pour son utilisation selon l'une quelconque des revendications précédentes, **caractérisé en ce qu'**il est choisi parmi :
- le sel de potassium du sucrose octasulfate ;
- le sel d'argent du sucrose octasulfate ; et
- le complexe hydroxyaluminium du sucrose octasulfate.

6. Oligosaccharide pour son utilisation selon l'une quelconque des revendications précédentes **caractérisé en ce que** ce soit le sel de potassium de sucrose octasulfate.

7. Oligosaccharide pour son utilisation selon l'une quelconque des revendications précédentes, **caractérisé en ce qu'**il est mis en œuvre sous la forme d'une composition telle qu'un gel, une solution, une émulsion, une crème, des granules ou des capsules permettant une application directement au niveau de la plaie.

8. Composition pharmaceutique comprenant un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, ledit oligosaccharide étant le sucrose octasulfate et lesdits grades étant définis dans le Tableau 1 :

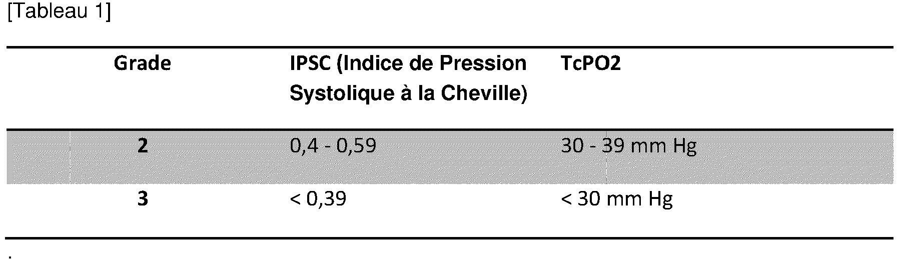

9. Pansement comprenant un oligosaccharide polysulfaté synthétique ayant 1 à 4 unités oses, ses sels, ou ses complexes, pour son utilisation pour augmenter l'oxygénation de la peau lors du traitement des plaies ischémiques de grade 2 et/ou 3, ledit oligosaccharide étant le sucrose octasulfate et lesdits grades étant définis dans le Tableau 1 :

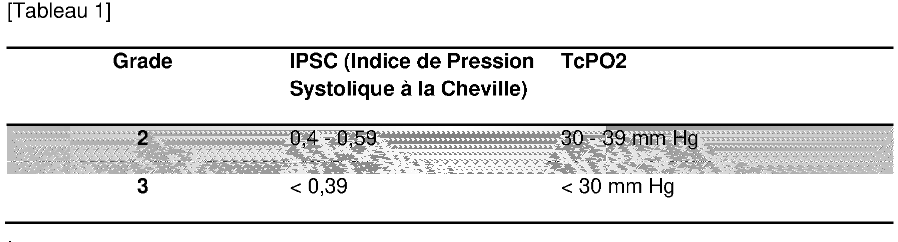

10. Pansement pour son utilisation selon la revendication 9, comprenant une trame textile enduite d'une matrice élastométique, ladite matrice comprenant ledit oligosaccharide polysulfaté synthétique.

## Patentansprüche

1. Synthetisches polysulfatiertes Oligosaccharid, umfassend 1 bis 4 Glykoseeinheiten, ihre Salze oder Komplexe zur Verwendung zur Erhöhung der Sauerstoffversorgung der Haut bei der Behandlung von ischämischen Wunden des Grades 2 und/oder 3, wobei das Oligosaccharid Sucroseoctasulfat ist und die Grade in Tabelle 1 definiert sind:
**[Tabelle 1]**
| **Grad** | **IPSC (Index für den systolischen Druck am Knöchel)** | **TcP02** |
|---|---|---|
| 2 | 0,4 - 0,59 | 30 - 39 mm Hg |
| 3 | < 0,39 | < 30 mm Hg |

2. Oligosaccharid zur Verwendung nach Anspruch 1, **dadurch gekennzeichnet, dass** die ischämischen Wunden aus arteriellen Beingeschwüren, gemischten Beingeschwüren, Dekubitusgeschwüren und neuroischämischen diabetischen Fußgeschwüren gewählt sind.

3. Oligosaccharid zur Verwendung nach einem der vorhergehenden Ansprüche, **dadurch gekennzeichnet, dass** die ischämischen Wunden aus arteriellen Beingeschwüren, gemischten Beingeschwüren und Dekubitusgeschwüren gewählt sind.

4. Oligosaccharid zur Verwendung nach einem der vorhergehenden Ansprüche, **dadurch gekennzeichnet, dass** seine Konzentration größer oder gleich 70 mg/ml, bevorzugt 100 mg/ml und stärker bevorzugt zwischen 100 und 1000 mg/ml beträgt.

5. Oligosaccharid zur Verwendung nach einem der vorhergehenden Ansprüche, **dadurch gekennzeichnet, dass** es gewählt ist aus:
- dem Kaliumsalz von Sucroseoctasulfat;
- dem Silbersalz von Sucroseoctasulfat; und
- dem Hydroxyaluminiumkomplex von Sucroseoctasulfat.

6. Oligosaccharid zur Verwendung nach einem der vorhergehenden Ansprüche, **dadurch gekennzeichnet, dass** es sich um das Kaliumsalz von Sucroseoctasulfat handelt.

7. Oligosaccharid zur Verwendung nach einem der vorhergehenden Ansprüche, **dadurch gekennzeichnet, dass** es in Form einer Zusammensetzung wie einem Gel, einer Lösung, einer Emulsion, einer Creme, Granulaten oder Kapseln zur direkten Anwendung auf die Wunde verwendet wird.

8. Pharmazeutische Zusammensetzung, umfassend ein synthetisches polysulfatiertes Oligosaccharid mit 1 bis 4 Glykoseeinheiten, dessen Salze oder Komplexe zur Verwendung zur Erhöhung der Sauerstoffversorgung der Haut bei der Behandlung von ischämischen Wunden des Grades 2 und/oder 3, wobei das Oligosaccharid Sucroseoctasulfat ist und die Grade in Tabelle 1 definiert sind:
**[Tabelle 1]**
| **Grad** | **IPSC (Index für den systolischen Druck am Knöchel)** | **TcP02** |
|---|---|---|
| 2 | 0,4 - 0,59 | 30 - 39 mm Hg |
| 3 | < 0,39 | < 30 mm Hg |

9. Verband, umfassend ein synthetisches polysulfatiertes Oligosaccharid, umfassend 1 bis 4 Glykoseeinheiten, dessen Salze oder Komplexe zur Verwendung zur Erhöhung der Sauerstoffversorgung der Haut bei der Behandlung von ischämischen Wunden des Grades 2 und/oder 3, wobei das Oligosaccharid Sucroseoctasulfat ist und die Grade in Tabelle 1 definiert sind:
**[Tabelle 1]**
| **Grad** | **IPSC (Index für den systolischen Druck am Knöchel)** | **TcP02** |
|---|---|---|
| 2 | 0,4 - 0,59 | 30 - 39 mm Hg |
| 3 | < 0,39 | < 30 mm Hg |

10. Verband zur Verwendung nach Anspruch 9, umfassend ein Textilgewebe, das mit einer elastomeren Matrix beschichtet ist, wobei die Matrix das synthetische polysulfatierte Oligosaccharid umfasst.

## Claims

1. Synthetic polysulfated oligosaccharide having 1 to 4 monosaccharide units, salts thereof, or complexes thereof, for use in increasing the oxygenation of the skin when treating grade 2 and/or 3 ischaemic wounds, said oligosaccharide being sucrose octasulfate and said grades being defined in Table 1:

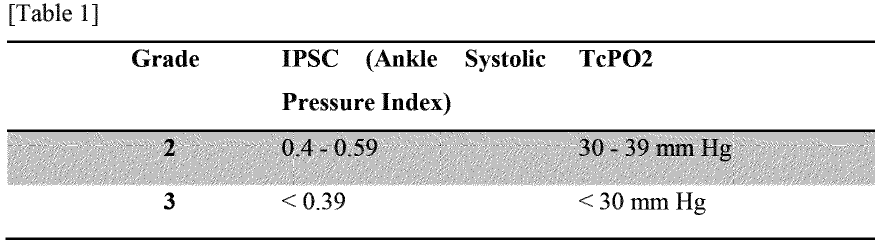

2. Oligosaccharide for use according to claim 1, **characterised in that** the ischaemic wounds are selected from arterial leg ulcer, mixed leg ulcer, pressure ulcer, and neuroischaemic diabetic foot ulcer.

3. Oligosaccharide for use according to any one of the preceding claims, **characterised in that** the ischaemic wounds are selected from arterial leg ulcer, mixed leg ulcer, and decubitus ulcer.

4. Oligosaccharide for use according to any one of the preceding claims, **characterised in that** its concentration is greater than or equal to 70 mg/mL, preferably 100 mg/mL, and more preferably between 100 and 1000 mg/mL.

5. Oligosaccharide for use according to any one of the preceding claims, **characterised in that** it is selected from:
- the potassium salt of sucrose octasulfate;
- the silver salt of sucrose octasulfate; and
- the hydroxyaluminium complex of sucrose octasulfate.

6. Oligosaccharide for use according to any one of the preceding claims, **characterised in that** it is the potassium salt of sucrose octasulfate.

7. Oligosaccharide for use according to any one of the preceding claims, **characterised in that** it is used in the form of a composition such as a gel, a solution, an emulsion, a cream, granules or capsules allowing direct application to the wound.

8. Pharmaceutical composition comprising a synthetic polysulfated oligosaccharide having 1 to 4 monosaccharide units, salts thereof, or complexes thereof, for use in increasing the oxygenation of the skin when treating grade 2 and/or 3 ischaemic wounds, said oligosaccharide being sucrose octasulfate and said grades being defined in Table 1:

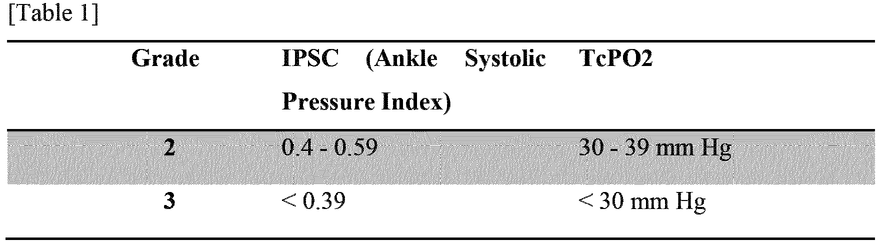

9. Dressing comprising a synthetic polysulfated oligosaccharide having 1 to 4 sugar units, salts thereof, or complexes thereof, for use in increasing the oxygenation of the skin when treating grade 2 and/or 3 ischaemic wounds, said oligosaccharide being sucrose octasulfate and said grades being defined in Table 1:

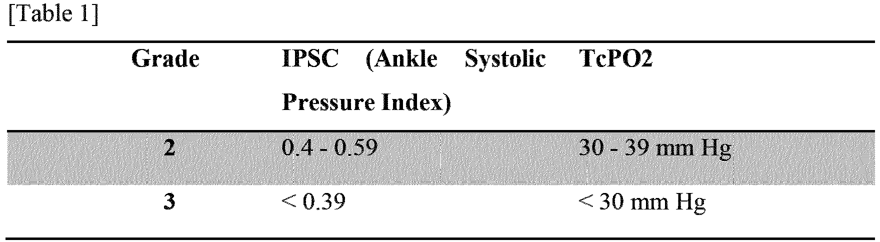

10. Dressing for use according to claim 9, comprising a textile weft coated with an elastomeric matrix, said matrix comprising said synthetic polysulfated oligosaccharide.
